# EUROPEAN PATENT SPECIFICATION

(11) **EP 4 646 024 B1**
(45) Date of publication and mention of the grant of the patent: **04.11.2026**
(21) Application number: 25203617.3
(22) Date of filing: 25.12.2014
(51) Int. Cl.: A24F 47/00, H05B 1/02

(54) **DYNAMIC OUTPUT POWER MANAGEMENT FOR ELECTRONIC SMOKING DEVICE**
DYNAMISCHES AUSGANGSLEISTUNGSMANAGEMENT FÜR EINE ELEKTRONISCHE RAUCHVORRICHTUNG
GESTION DYNAMIQUE DE PUISSANCE DE SORTIE POUR DISPOSITIF À FUMER ÉLECTRONIQUE

(43) Date of publication of application: 05.11.2025
(60) Divisional application: 26200173.8 / 4 819 761
(62) Document number(s) of the earlier application(s) in accordance with Art. 76 EPC: 23150527.2 / 4 183 282; 14908768.6 / 3 236 787
(73) Proprietor: Fontem Ventures B.V., 1043 NT Amsterdam (NL)
(72) Inventor: Hon, Lik, Beijing 100020 (CN); Li, Zhuoran, Beijing 100007 (CN)
(74) Representative: Gulde & Partner

(56) References cited:
- DE-U1- 202013 010 986
- US-A1- 2014 270 727
- US-A1- 2014 278 250
- US-A1- 2014 334 804

## Description

### TECHNICAL FIELD

The present invention relates to an output power management unit (PMU) for electronic smoking devices.

### BACKGROUND OF THE INVENTION

An electronic smoking device, such as an electronic cigarette, usually has a heating element for vaporizing liquid and a power supply for providing power to the heating element. In some cases, the heating element can be a component of an atomizer. Providing a consistent energy supply to the heating element improves the consistency of each puff. Each puff on average takes about the same time (e.g. 2.5 seconds/puff). A consistent energy supply to the heating element for each puff may be achieved via consistent power output of the power supply during this interval.

Typically, the power supply is a disposable or rechargeable battery with working voltage decreasing over its useful life. The decreasing voltage may result in inconsistent puffs. Moreover, the heating elements may have resistances that vary in operation due to factors, such as the amount of e-solution, the heating element contacts, and the operating temperature.

Therefore, there is a need for a dynamic output power management unit to provide a stable output power in response to the changing capacity of the battery, and/or the changing resistance of the heating element.

US 2014/334804 A1 discloses a control unit for a portable atomizing apparatus that prevents overheating of the heating element. Instead of continuous power, the control unit provides a pulsed output signal to the heater during a puff. This signal alternates between a heating time section at a first voltage level and a heating-stopped time section at a lower (or zero) voltage level. The user may be able to adjust the durations of these sections via a user interface.

US 2014/270727 A1 discloses a method and apparatus for precisely controlling the power delivered to the heating element of an electronic smoking article. It uses a processor-based feedback loop to maintain a constant power output.

DE 20 2013 010986 U1 discloses an electronic cigarette with power electronics designed to provide a consistent heating performance. The user sets a desired heating power via a data input device. The power electronics then regulate the energy delivered to the heating element to maintain this desired power level, specifically compensating for disturbances such as changes in the heater's ohmic resistance and decreases in the power source's voltage over time.

US 2014/278250 A1 discloses a system and method for obtaining smoking topography data from an electronic smoking article. The core concept involves using a processor to detect a smoking event (e.g., a puff) and measuring the shift in battery voltage that occurs between the start and end of that event.

### BRIEF DESCRIPTION OF THE DRAWINGS

Fig. 1 is a schematic drawing of an electronic cigarette or similar vaporizing device.
Fig.1A is a diagram showing a heating circuit of an electronic cigarette including a dynamic output power management unit;
Fig.1 B is a diagram showing another embodiment of a heating circuit of an electronic cigarette including a dynamic output power management unit;
Fig.2 is a diagram showing the discharging time of a power supply when the heating element has a constant resistance;
Fig.3 is a diagram showing the discharging time of a power supply when the heating element has a variable resistance;
Fig.4 is a diagram showing the discharging time of another power supply when the heating element has a variable resistance;
Fig.5 is a block diagram illustrating the dynamic output power management unit in Fig.1A;
Fig.6 is a block diagram illustrating the dynamic output power management unit in Fig.1B;
Fig.7A is a flowchart of a control method by the power management unit illustrated in Fig.5;
Fig.7B is a flowchart of a control mechanism implemented by the power management unit illustrated in Fig.5 according another embodiment of the invention;
Fig.8A is a flowchart of an alternative control method by the power management unit illustrated in Fig.6;
Fig.8B is a flowchart of an alternative control method by the power management unit illustrated in Fig.6; and
Fig.9 is a block diagram illustrating another example of the dynamic output power management unit in Fig.1B.

### DETAILED DESCRIPTION

A dynamic output power management unit for an electronic cigarette, cigar pipe etc. provides a substantially constant amount of vaporized liquid in a predetermined time interval, for example, the duration of one puff. This can increase compatibility of an electronic cigarette to various types of heating elements, and /or may compensate for dropping output voltage of the power source.

For conventional electronic cigarettes, a heating circuit typically includes a heating element connected to the power source via a switching element which is turned on when a puff is detected and is still on for a constant time of time for each puff.in contract, with the present PMU the discharging time of the power source is adjusted dynamically to obtain more consistent vaporization over the same time interval. Consequently a more consistent amount of aerosol may be inhaled by a user during each puff.

To compensate for an output voltage of the power source which drops over the discharging time, waveform control techniques, for example, PWM (pulse width modulation) technique maybe used to control at least one switching element within the heating circuit, to control the active time of the heating circuit. A waveform generator can be used to generate the desired control waveform. The waveform generator can be a PWM waveform generator within a PWM controller or PWM module in a microcontroller, for example, a MOSFET. A high-time and low-time ratio is determined, which is then used by the PWM controller for controlling the ON/OFF switching of the heating circuit.

In designs where the resistance of the heating element changes as the working temperature changes, the instantaneous resistance of the heating element may be measured in real-time by incorporating a reference component, for example a reference resister, into the heating circuit to control the active time of the heating circuit.

Changing resistance of the heating element may change the amount of aerosol generated during the process of vaporization, resulting in variation in the amount of the resulting in variations in the amount or character of the vapor generated, the nicotine for example, need to be controlled within a particular range so that human being's throat will not be irritated or certain administrative regulatory requirements could be meet. Therefore, another benefit of the dynamic output power management technique is that it can be compatible to various types of heating elements, for example, metal coils and heating fiber among others. Especially for heating element made from fibers, carbon fiber bundles for example, of which a precise resistance cannot be feasibly maintained for all the carbon fiber bundles in a same batch, the dynamic output management technique is desirable since it can adjust the output power within a range in responsive to carbon fiber bundles with resistance within a range of, for example 1.5 ohms. This would alleviate the burden of the manufacturing process of the carbon fiber bundle and lower the cost of the carbon fiber bundles as a result.

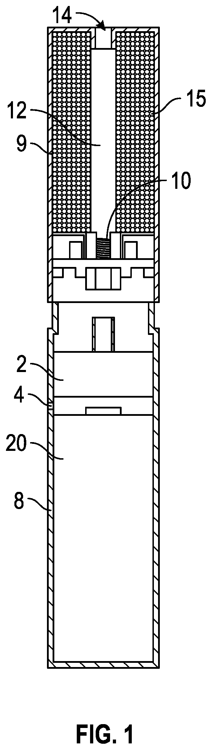

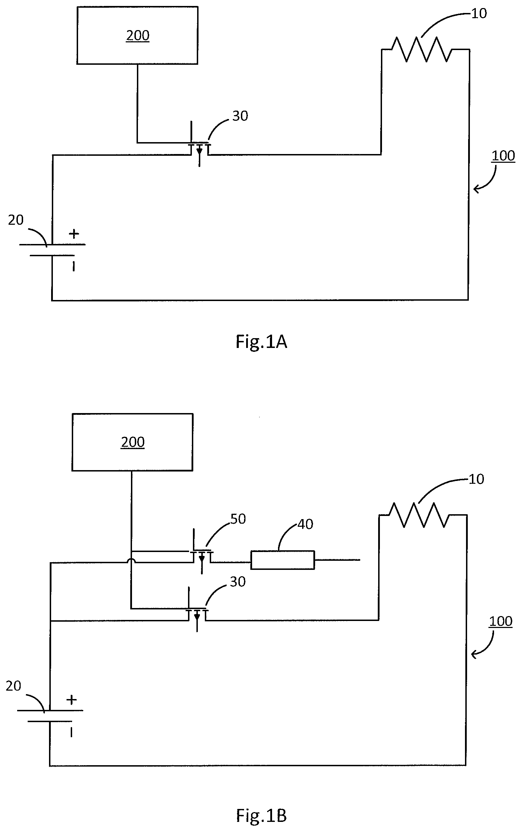

Fig. 1 shows an example of an electronic cigarette which may have a size and shape generally comparable to a real tobacco cigarette, typically about 100 mm with a 7.5 mm diameter, although lengths may range from 70 to 150 or 180 mm, and diameters from 5 to 20 mm. A power source or battery 20 is contained within the electronic cigarette housing, which is optionally divided into a first housing 8 and a second housing 9. One or more inlets 4 are provided in the housing, and an outlet 14 is located at the back end of the electronic cigarette. An electronic controller 2 including the PMU is electrically connected to the battery and to the heating element 10, which may be in the form of a wire coil. A liquid holding container or space 15 may surround a channel 12 extending from the heating element 10 to the outlet 14.

Referring to Fig.1A, a heating circuit 100 includes the heating element 10, the power source 20, and a switching element 30 connected between the heating element 10 and the power source 20. The heating coil can be treated or remain substantially dry during working so that it has a substantially constant resistance at the working temperature range. The first switching element 30 can be a first MOSFET switch, which is configurable between an ON state and an OFF state by a first control waveform. The power source 20 can be a common battery, for example, a Nickel-Hydrogen rechargeable battery, a Lithium rechargeable battery, a Lithium-manganese disposable battery, or a zinc- manganese disposable battery. The first control waveform can be generated by a waveform generator which can be included in the power management unit 200 or can be implemented by a dedicated circuitry or by a processor or a controller implementing functions.

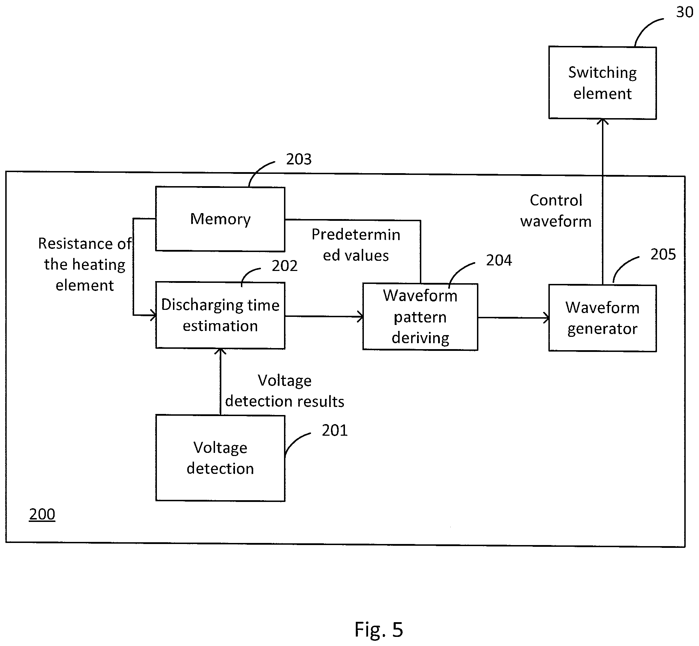

Fig.5 shows an alternative embodiment where the PMU 200 has at least one voltage detector 201 for detecting output voltage of the power source 20. A discharging time estimation device 202 estimates the discharging time of the power source in the duration of a puff based on the output voltage detected and a resistance of the heating element stored in a memory device 203. A waveform pattern deriving device 204 determines the hightime and lowtime ratio of the first control waveform based on the estimated discharging time and a predetermined power consumption P and a time a puff normally lasts tₚ stored in the memory. A waveform generator 205 generates first control waveform according to the pattern determined.

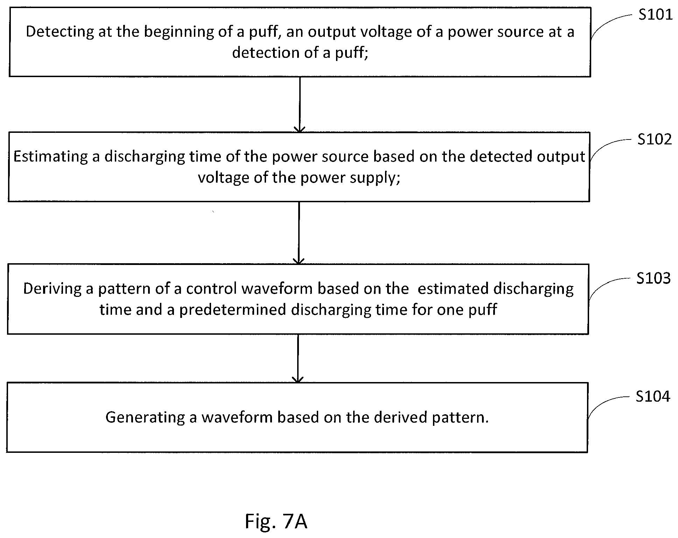

As illustrated in Fig.7A, at step S101 detection of the working voltage of the power supply can be done at the beginning of each puff to derive the time the heating element should be powered. The predetermined power consumption P and the time a puff normally lasts tₚ are known parameters and can be stored in advance within the memory device 203, for example, registers within a microcontroller.

The energy consumption of the heating element for one puff is estimated based on the resistance of the heating element using Equation 1, which is then used at step S102 for deriving a period of time that needed for providing the heating element with the desired energy: Equation 1: $P X {t}_{p} / {t}_{h - p} = {V}^{2} / {R}_{h} or {t}_{h - p} / {t}_{p} = P X {R}_{h} / {V}^{2} ;$ wherein P is a predetermined power consumption of the heating element for one puff; tₕ₋ₚ is the time of the heating element should be powered on; tₚ is the time a puff normally last; V is the working voltage of the power supply; and Rₕ is the resistance of the heating element.

With the estimated time that the heating element is to be powered, at step S103 a waveform pattern can be derived.

For example, the derived tₕ₋ₚ can be equal to or greater than the duration of a puff tₚ. In this circumstances, the first MOSFET switch 30 can be maintained at the OFF state during the entire puff duration. The output of the power source 20 that applied onto the heating element 10 in this puff then presents in the form of a DC output.

In other examples, the derived tₕ₋ₚ can be smaller than the duration of each puff tₚ. In this case, the first MOSFET switch 30 can be configured according to different control waveforms of different hightime and lowtime ratios, to reflect the ratio of tₕ₋ₚ to tₚ.

A waveform device, for example the waveform generator 205 is then used at step S104 to generate the first control waveform according to the derived waveform pattern.

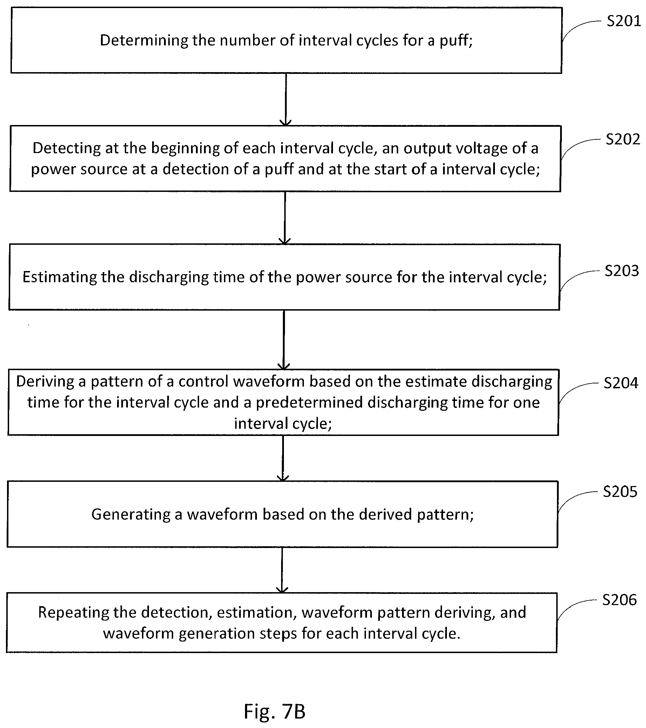

In a further embodiment, as illustrated in Fig.7B, a puff can be divided into multiple interval cycles, for example N interval cycles, each cycle t_{c} will last for a time of t_{c} =tₚ/N, S201. Working voltage of the power source can be slightly different in the respective interval cycles and discharging time of the power source for each interval cycle can be derived accordingly based on detection of the working voltage at the beginning of each interval cycle S202. Similar algorithms as described above can be applied to each cycle to determine the time the heating element should be powered for the duration of t_{c} The time of the heating element should be powered for each cycle t'ₕ₋ₚ can be derived at step S203 from Equation 2: $t {′}_{h - c} / {t}_{c} = P X {R}_{h} / {V}^{2} ;$ wherein P is a predetermined power consumption of the heating element for one interval cycle, and the predetermined power consumption for one interval cycle can be a result of the predetermined power consumption for a cycle divided by the number of interval cycles.

Similarly, with the estimated time that the heating element is to be powered at step S204, a waveform pattern can be derived.

The derived t'ₕ₋ₚ can be equal to or greater than the duration of an interval cycle t_{c}. The first MOSFET switch 30 can thus be maintained at the OFF state during the entire interval cycle. The output of the power source 20 applied to the heating element 10 in this interval cycle is in the form of a DC output.

In other examples, the derived t'ₕ₋ₚ can be smaller than the duration of each puff t_{c}, and the first MOSFET switch 30 can be configured according to different control waveforms of different hightime and lowtime ratios, to reflect the ratio of t'ₕ₋ₚ to t_{c}. In accordance with this step, energy converted in a period of time is substantially identical to a predetermined energy conversion value for a same period of time.

A waveform device, for example the waveform generator 205 is then used in step S205 to generate the first control waveform according to the derived waveform pattern. The process is repeated until waveforms for all interval cycles of the puff are generated. Bipolar transistors and diodes can also be used as switching elements for activating or deactivating the heating circuit instead of using MOSFET's as switching elements

The first control waveform can be a PWM (Pulse Width Modulation) waveform and the waveform generator can be a PWM waveform generator. The PWM waveform generator can be part of a microprocessor or part of a PWM controller.

The design in Fig.1 B includes the elements of Fig.1A and further includes a reference element 40, for example a reference resistor or a set of reference resistors connected in series or in parallel and having a substantially constant resistance value. The reference element 40 is connected in series with the heating element 10 and disconnected from the heating circuit via a second switching element 50, for example a second MOSFET switch which is configurable between an ON state and an OFF state by a second control waveform. The reference resistor 40 has a known resistance R_{f} that is consistent over the working temperature and working time of the electronic cigarette.

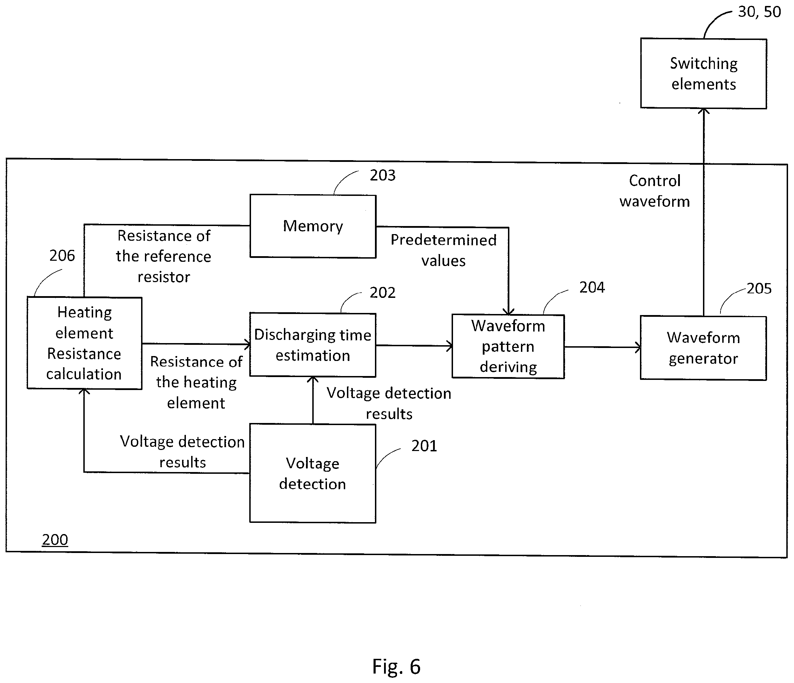

A block diagram of the power management unit 200 of Fig.1B is illustrated in Fig.6. The unit 200 has at least one voltage detector 201 for detecting an output voltage of the power source 20 and/or a voltage drop across the reference resistor, and/or a voltage drop across the heating element. A heating element resistance calculation unit 206 calculates the instantaneous resistance or mean value of the resistance of the heating element based on the detected output voltage of the power source and/or the voltage drop across the reference resistor and/or the voltage drop across the heating element, and a resistance value of the reference resistor stored within a memory device 203. A discharging time estimation device 202 estimates the discharging time of the power source in the duration of a puff based on the output voltage detected and the calculated resistance of the heating element. A waveform pattern deriving device 204 determines the hightime and lowtime ratio of the first control waveform based on the estimated discharging time and a predetermined power consumption P and a time a puff normally lasts tₚ stored in the memory 203. A waveform generator 205 generates the first control waveform according to the pattern determined.

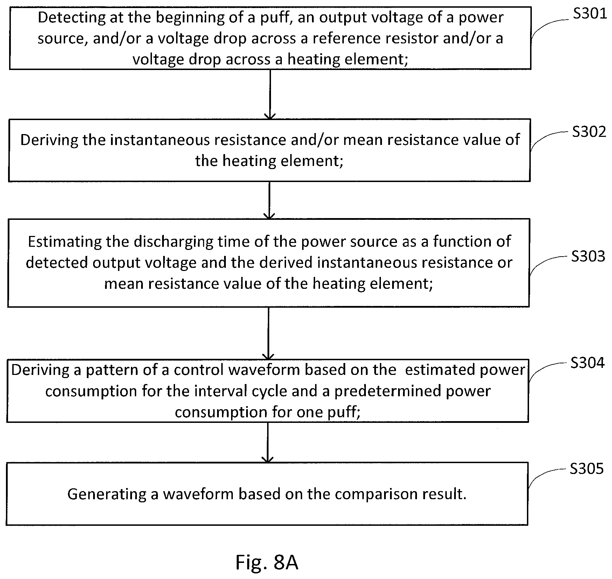

To detect an output voltage of a power source, and/or a voltage drop across a reference resistor and/or a voltage drop across a heating element 10, the first MOSFET switch 30 is configured to the ON state and the second MOSFET switch 50 is configured to the OFF state. The power source 20, the reference resistor 40 and the heating element 10 are connected as a closed circuit. As illustrated in Fig.8A, at step S301 detection of the working voltage of the power source 20 and/or the voltage drop across the heating element 10 are performed. The instantaneous resistance can then be derived at step S302 by calculating with reference to the resistance of the reference resistor 40 and the voltages measured using Equation 3: ${R}_{h} = {V}_{2} X {R}_{f} / \left({V}_{1}-{V}_{2}\right) ;$ wherein Rₕ is the instantaneous resistance of the heating element; R_{f} is the resistance of the reference resistor; V₁ is the working voltage of the DC power source; and V₂ is the voltage drop across the heating element.

Alternatively or in addition, at step S302 voltage drop across the reference resistor 40 can be detected for deriving the instantaneous resistance of the heating element 10. Equation 3 can in turn be slightly adjusted to involve the voltage drop of the reference resistor 40 instead of the output voltage of the power source 20.

The measurement and calculation of the instantaneous resistance of the heating element can be repeated, and a mean value of can be derived from the result of the repeated calculation results and can be used for further processing.

After the instantaneous resistance or the mean resistance of the heating element is calculated. An output voltage of the power source 20 is detected again with the first MOSFET switch 30 in the OFF state and the second MOSFET switch in the ON state. A discharging time of the power source for one puff is then estimated at step S303 based on the calculated resistance of the heating element and the newly detected output voltage of the power source using Equation 1. After the discharging time is estimated, at step S304 a waveform pattern can be determined and control waveforms can be generated at step S305.

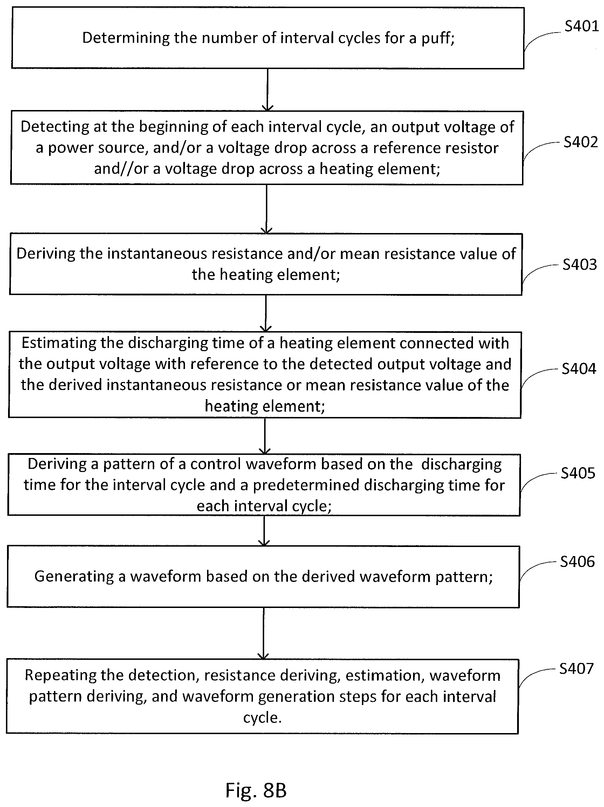

Likewise, in this embodiment, as illustrated in Fig.8B, a puff can also be divided into multiple interval cycles, for example N interval cycles, each cycle t_{c} lasting for a time of t_{c} =tₚ/N, S401. Equation 2 can again be used to derive the time of the heating element that should be powered for each cycle.

At a beginning of a first time interval, the first MOSFET switch 30 is ON and the second MOSFET switch 50 is OFF. Voltage drop across the reference resistor 40 and the output voltage of the power source are then detected at step S402. The instantaneous resistance of the heating element 10 can then be derived from Equation 3 at step S403.

After the instantaneous resistance of the heating element is derived, the first MOSFET switch 30 is configured to the OFF state and the second MOSFET resistor 50 is configured to the ON state whereby the reference resistor 40 is disconnected from the heating circuit 100. The output voltage V of the power source 20 is then detected again and the discharging time of the power source 20, that is, the time that the first MOSFET switch 30 needs to be maintained at the OFF state in the interval cycle for a desired energy conversion at the heating element, is derived according to Equation 2 at step S404.

The time that the first MOSFET switch 30 should be maintained at the OFF state is then derived for each interval cycle following the same process as mentioned above. In some embodiments, the instantaneous resistance of the heating element is derived at the beginning of each puff and is only derived once and is then used for deriving the time that the first MOSFET switch 30 should be at the OFF state for the duration of the puff. In other embodiments, the instantaneous resistance of the heating element 10 is derived at the beginning of each interval cycle and is used only for deriving the time that the first MOSFET switch 30 needs to be maintained at the OFF state for that interval cycle. Deriving the instantaneous resistance of the heating element may be desirable if the heating element is very sensitive to its working temperature.

Similarly, a mean value of the resistance for the reference resistor can be derived instead and used for deriving the time that the first MOSFET switch 30 needs to be configured at the OFF state.

In some embodiments, the derived t'ₕ₋ₚ can be equal to or greater than the duration of each interval cycle t_{c}. In this case, the first MOSFET switch 30 will be maintained at the OFF state during the entire interval cycle and based on the ratio of t'ₕ₋ₚ to t_{c}, the first MOSFET switch 30 may also be maintained at the OFF state for a certain period of time in a subsequent interval cycle or the entire duration of the subsequent interval cycle. The power source 30 supplies a DC output current to the heating element 10 in this interval cycle or interval cycles.

In other embodiments, the derived t'ₕ₋ₚ can be smaller than the duration of each interval cycle tc. Then, the first MOSFET switch 30 is configured according to different control waveforms, for example PWM waveforms of different high time and low time ratios, to reflect the ratio of t'h-p to tc.

For example, at step S405 a waveform pattern is then determined according to the ratio of t'ₕ₋ₚ to t_{c} and the first and the second control waveforms are generated according to the determined waveform pattern at step S406. Control waveforms for all interval cycles are generated by repeating the above steps at step S407. Similar to the first control waveform, the second control waveform can also be a PWM waveform and the waveform generator can be a PWM waveform generator. The PWM waveform generator can also be part of a microprocessor or part of a PWM controller.

Alternatively or in addition to the embodiment described in Fig.1B, the reference resistor 40 can be arranged in parallel with the heating element 10. In this arrangement, the instantaneous resistance of the heating element 10 can be derived with reference to the current flow across each branch of the heating circuit. In some embodiments, the voltage across the reference resistor 40 and the heating element 10 can be detected by a voltage probe, a voltage measurement circuit, or a voltage measurement device.

Calculations according to Equations 1 to 3 can be performed by a processor or a controller executing instruction codes or by dedicated calculation circuits designed to perform the above mentioned logic. A microprocessor having a PWM function and a storage function may be used. The storage function can store the instructions code that when executed by the microprocessor can implement the logic as described above.

In a further embodiment, instead of deriving the discharging time to generate the control waveforms, an estimated power consumption of the heating element can be derived for generating the control waveforms.

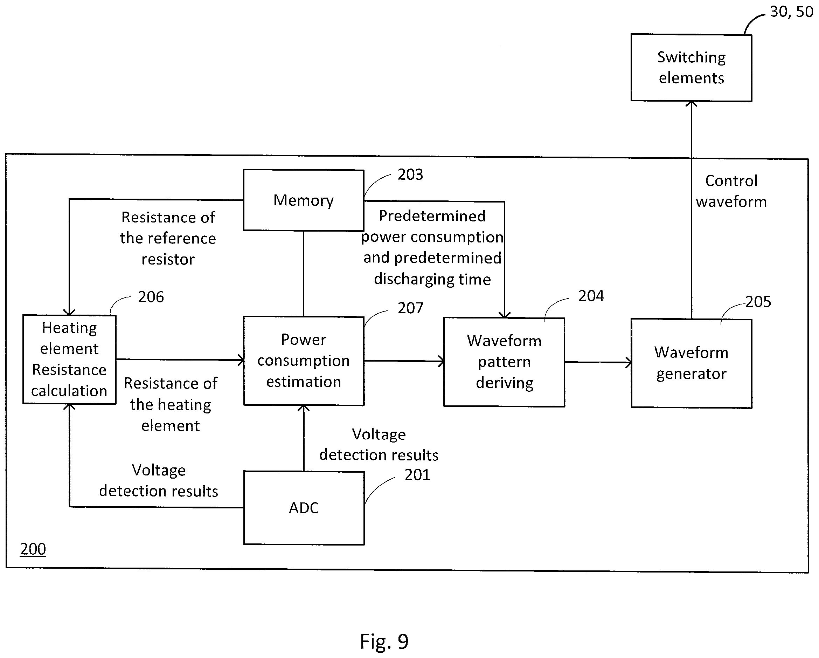

**As illustrated** in Fig.9, the power management unit in this example includes an ADC 201 for detecting a first output voltage of the power source 20 and/or a voltage drop across the reference resistor 40, and/or a voltage drop across the heating element 10. A heating element resistance calculation unit 206 calculates the instantaneous resistance or mean value of the resistance of the heating element based on the detected first output voltage of the power source and/or the voltage drop across the reference resistor and/or the voltage drop across the heating element. A resistance value of the reference resistor 40 stored within a memory device 203. A power consumption estimation device 207 estimates the power consumption during a given period of time, for example the duration of a puff or an interval cycle within the puff, based on a second output voltage detected and the calculated resistance of the heating element. A waveform pattern deriving device 204 determines the hightime and lowtime ratio of the first control waveform based on the estimated power consumption and a predetermined power consumption P stored in the memory 203. A waveform generator 205 generates a first control waveform according to the pattern determined.

The heating element in this example may be a carbon fiber based heating element. An ADC of a microcontroller reads the voltage ratio of the carbon fiber heating element V_{Wick} and the voltage drop *Vᵣₑₛ* across a reference resistor having a resistance of R_{standard}. The resistance of the standard resistor is known, and the resistance of the carbon fiber heating element can be derived by Equation 4: ${R}_{wick} = \left({V}_{wick}-{V}_{res}\right) / {R}_{standard}$

The reference resistor is then disconnected from the heating circuit and the carbon fiber heating element. The ADC then reads the closed circuit voltage of the carbon fiber V_{close}. The power of the carbon fiber can be calculated by Equation 5:${P}_{CF} = {V}_{close}^{2} / {R}_{wick}$

The estimated power P_{CF} can be for example 3.2W which is higher than a predetermined value of 2.5W, the ON and OFF time of the first MOSFET switch 30 can then be determined by determining the hightime and lowtime ratio of the control waveform.

For example, in every 50ms long cycles, the hightime is 50ms * *hightime*/*lowtime* = 50ms * 0.78 = 39ms, the lowtime is 50ms *- hightime =* 11*ms.*

A control waveform is then generated by the waveform generator to configure the ON/OFF time of the first MOSFET switch 30.

In case the estimated P_{CF} is smaller than the predetermined value of 2.5W, the output waveform to the first MOSFET controller will be all OFF, and the output of the power source will be provided as DC.

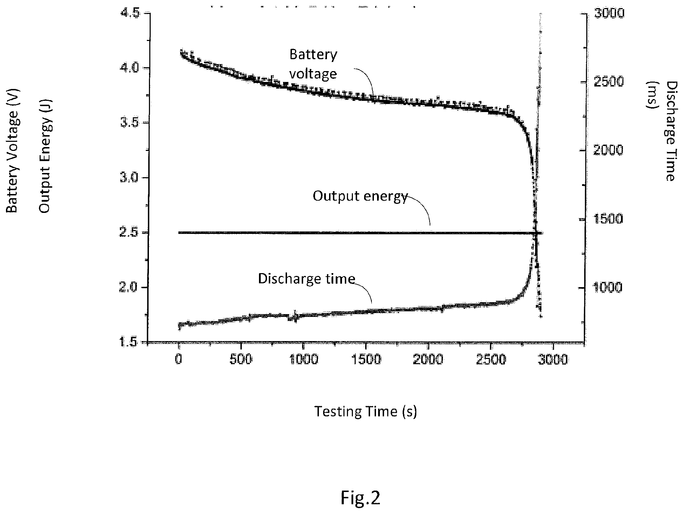

Figs. 2 to 4 are diagrams showing testing results of the heating circuit using the power management unit. These results shows substantially constant output have been maintained even though the resistance of the heating element may vary during the working cycle of the heating element and/or the battery voltage may drop with the lapse of time.

### Testing result 1: Substantially constant resistance of the heating element with decreasing battery capacity

In one example, dynamic discharging tests using the dynamic output power management unit of Fig.1A were carried out on a dry heating element, i.e., a heating element having substantially consistent resistance. The results are shown in Fig.2, wherein the data lines from the top to the bottom represent the battery voltage V, the output energy in J at 280 mAh, and the discharge time in ms, i.e. the powered time, over testing time in seconds.

In some examples, the resistance of the heating element changes depending on the working condition of the heating element, e.g. amount of e-solution the heating element contacts, carbonization around/in the heating element, and the working temperature. The heating element may be a conventional heating element or a fiber based heating element, for example a carbon fiber heating element.

### Example 2: Wetted heating element with decreasing battery capacity

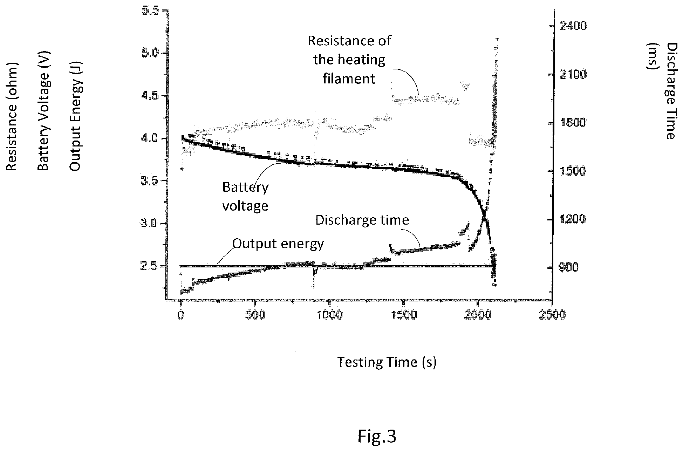

In another example, wet dynamic discharging tests using the dynamic output power management unit of Fig.1A or 1B were carried out on a wetted heating element, i.e., the resistance of the heating element may change when it has different amount of liquid. The results are shown in Fig.3. The data lines from the top to the bottom represent the resistance of the heating element in ohms, the battery voltage V, the output energy in J, at 240 mAh, and the discharge time in ms, i.e. the powered time, over testing time in seconds.

### Example 3: Wetted heating element with decreasing battery capacity

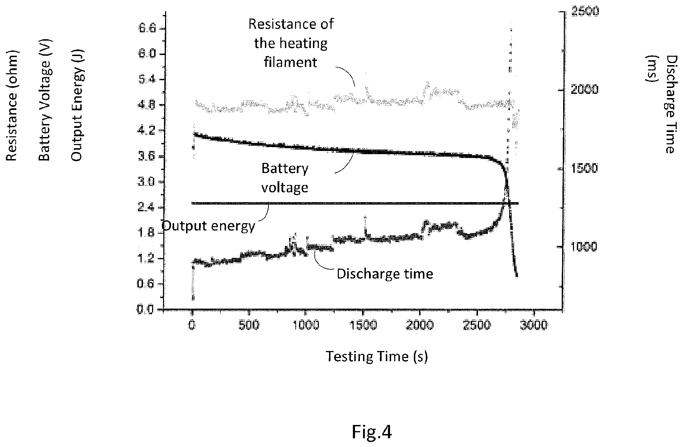

The results for another set of wet dynamic discharging tests are shown in Fig.4. The data lines from the top to the bottom represent the resistance of the heating element in ohms, the battery voltage V, the output energy in J at 280 mAh, and the discharge time in ms, i.e. the powered time, over testing time in seconds.

The power management system described may include dynamic output power management unit for a heating circuit of an electronic smoking device, with the PMU having at least one voltage detection device to detect an output voltage of a power source, and/or a voltage drop across a heating element operable to be connected to or disconnected from the power source via a first switching element, and/or a voltage drop across a reference element operable to be connected to or disconnected from the heating circuit via a change of state of a second switching element from a first state to a second state and from a second state to the first state. A controller is configured to change the second switching element from the first state to the second state; to receive a first detection result from the detection device; derive a resistance of the heating element; change the second switching element from the second state to the first state; receive a second detection result from the voltage detection device; and derive a discharging time of the power source as a function of the resistance of the heating element and the second voltage detection. As a result, energy converted in a period of time is substantially identical to a predetermined energy conversion value for a same period of time.
The power management system described may operate on instructions stored on non-transitory machine-readable media, the instructions when executed causing a processor to control a voltage detection device to detect a first output voltage of a power source, and/or a voltage drop across a heating element operably connected to the power source via a first switching element, and/or a voltage drop across a reference element operably connected to the power source via a second switching element. The first output voltage is detected when the reference element is connected to the power source. The instructions may direct the processor to derive a resistance of the heating element as a function of the at least two of the first output voltage of a power source, the voltage drop across the heating element and the voltage drop across the heating element, and to control the voltage detection device to detect a second output voltage of the power source. The processor may then estimate the discharging time of the power source for the puff as a function of the second output voltage of the power source and the derived resistance of the heating element such that an energy converted in the puff is substantially identical to a predetermined energy conversion value for one puff.

## Claims

1. An electronic smoking device comprising:
an atomizer including an electrical resistance heating element (10);
a power source (20) connected to the heating element (10) and to a controller;
a first switching element (30) connected between the heating element (10) and the power source (20);
a voltage detection device (201) connected to the controller and to the power source (20) for measuring output voltage of the power source (20);
a memory (203) for providing the resistance of the heating element (10) configured for storing a resistance value; and
wherein the controller is configured to adjust electrical power supplied from the power source (20) to the heating element (10)
based on power source voltage and the provided resistance of the heating element (10).

2. The electronic smoking device of claim 1, further comprising: a reference element (40) and a second switching element (50) for switching the reference element (40) into and out of a series connection with the heating element (10).

3. The electronic smoking device of claim 1 or 2, wherein the controller is configured to estimate a discharging time of the power source (20) as a function of the measured output voltage of the power source (20) and the provided resistance of the heating element (10) such that an energy converted in a puff is substantially identical to a predetermined energy conversion value for one puff.

4. The electronic smoking device of claim 1 or 2, wherein a discharging time estimation device (202) is configured to estimate a discharging time of the power source (20) as a function of the measured output voltage of the power source (20) and the provided resistance of the heating element (10) such that an energy converted in a puff is substantially identical to a predetermined energy conversion value for one puff.

## Patentansprüche

1. Elektronische Rauchvorrichtung, umfassend:
einen Zerstäuber mit einem elektrischen Widerstandsheizelement (10);
eine Leistungsquelle (20), die mit dem Heizelement (10) und einer Steuerung verbunden ist;
ein erstes Schaltelement (30), das zwischen dem Heizelement (10) und der Leistungsquelle (20) geschaltet ist;
eine Spannungserfassungsvorrichtung (201), die mit der Steuerung und der Leistungsquelle (20) verbunden ist, um die Ausgangsspannung der Leistungsquelle (20) zu messen;
einen Speicher (203) zur Bereitstellung des Widerstands des Heizelements (10), der zum Speichern eines Widerstandswerts konfiguriert ist; und
wobei die Steuerung dazu konfiguriert ist, die von der Leistungsquelle (20) an das Heizelement (10) gelieferte elektrische Leistung
auf der Grundlage der Leistungsquellenspannung und des bereitgestellten Widerstands des Heizelements (10) anzupassen.

2. Elektronische Rauchvorrichtung nach Anspruch 1, umfassend ferner: ein Referenzelement (40) und ein zweites Schaltelement (50) zum Ein- und Ausschalten des Referenzelements (40) in bzw. aus einer Reihenschaltung mit dem Heizelement (10).

3. Elektronische Rauchvorrichtung nach Anspruch 1 oder 2, wobei die Steuerung dazu konfiguriert ist, eine Entladezeit der Leistungsquelle (20) als eine Funktion der gemessenen Ausgangsspannung der Leistungsquelle (20) und des bereitgestellten Widerstands des Heizelements (10) zu schätzen, so dass eine bei einem Zug umgewandelte Energie im Wesentlichen identisch mit einem vorbestimmten Energieumwandlungswert für einen Zug ist.

4. Elektronische Rauchvorrichtung nach Anspruch 1 oder 2, wobei eine Entladezeit-Schätzvorrichtung (202) dazu konfiguriert ist, eine Entladezeit der Leistungsquelle (20) als eine Funktion der gemessenen Ausgangsspannung der Leistungsquelle (20) und des bereitgestellten Widerstands des Heizelements (10) zu schätzen, so dass eine bei einem Zug umgewandelte Energie im Wesentlichen identisch mit einem vorbestimmten Energieumwandlungswert für einen Zug ist.

## Revendications

1. Dispositif à fumer électronique comprenant :
un atomiseur comprenant un élément chauffant à résistance électrique (10) ;
une source d'alimentation électrique (20) connectée à l'élément chauffant (10) et à un contrôleur ;
un premier élément de commutation (30) connecté entre l'élément chauffant (10) et la source d'alimentation électrique (20) ;
un dispositif de détection de tension (201) connecté au contrôleur et à la source d'alimentation électrique (20) pour mesurer une tension de sortie de la source d'alimentation électrique (20) ;
une mémoire (203) destinée à fournir la résistance de l'élément chauffant (10), configurée pour stocker une valeur de résistance ; et
dans lequel le contrôleur est configuré pour ajuster une puissance électrique fournie par la source d'alimentation électrique (20) à l'élément chauffant (10)
sur la base de la tension de la source d'alimentation électrique et de la résistance fournie de l'élément chauffant (10).

2. Dispositif à fumer électronique selon la revendication 1, comprenant en outre : un élément de référence (40) et un second élément de commutation (50) destiné à commuter l'élément de référence (40) dans une connexion en série avec l'élément chauffant (10) et hors de cette connexion en série.

3. Dispositif à fumer électronique selon la revendication 1 ou 2, dans lequel le contrôleur est configuré pour estimer une durée de décharge de la source d'alimentation électrique (20) en fonction de la tension de sortie mesurée de la source d'alimentation électrique (20) et de la résistance fournie de l'élément chauffant (10), de telle sorte qu'une énergie convertie au cours d'une bouffée est sensiblement identique à une valeur prédéterminée de conversion d'énergie pour une bouffée.

4. Dispositif à fumer électronique selon la revendication 1 ou 2, dans lequel un dispositif d'estimation de durée de décharge (202) est configuré pour estimer une durée de décharge de la source d'alimentation électrique (20) en fonction de la tension de sortie mesurée de la source d'alimentation électrique (20) et de la résistance fournie de l'élément chauffant (10), de telle sorte qu'une énergie convertie au cours d'une bouffée est sensiblement identique à une valeur prédéterminée de conversion d'énergie pour une bouffée.
